# EUROPÄISCHE PATENTSCHRIFT

(11) **EP 0 500 518 B1**
(45) Veröffentlichungstag und Bekanntmachung des Hinweises auf die Patenterteilung: **24.01.1996**
(21) Anmeldenummer: 92890039.8
(22) Anmeldetag: 18.02.1992
(51) Int. Cl.: B09B 3/00, A62D 3/00, C12N 1/26, C12S 1/00, C12N 1/00

(54) **Verfahren zum Regenerieren von mit Erdölprodukten kontaminierten Substraten**
Process for the regeneration of substrates contaminated with earth oil products
Procédé pour la regénération de substrats contaminés avec des produits de pétrole

(30) Priorität: 19.02.1991 AT 347/91
(43) Veröffentlichungstag der Anmeldung: 26.08.1992
(73) Patentinhaber: OMV Aktiengesellschaft, A-1091 Wien (AT)
(72) Erfinder: Ploder, Werner, Dipl.-Ing. Dr. techn., A-2320 Schwechat (AT)
(74) Vertreter: Widtmann, Georg, Dipl.-Ing. Dr. techn.

(56) Entgegenhaltungen:
- EP-A- 0 018 348
- EP-A- 0 436 493
- WO-A-88/01607
- DD-C- 139 083
- DE-A- 2 404 562
- GB-A- 649 818
- US-A- 4 119 495
- US-A- 4 439 523

## Beschreibung

Die Erfindung bezieht sich auf ein Verfahren zum Regenerieren von Mikroorganismen aufweisendem Substrat.

Bei Raumtemperatur flüssige Erdölfraktionen sind für die Versorgung von stationären und mobilen Energieverbrauchern von hoher Bedeutung. Die Zufuhr dieser Erdölfraktionen erfolgt entweder über Rohrleitungen oder über Tankwagen, die entweder auf der Straße oder auch auf der Schiene transportiert werden. Trotz hoher Sorgfalt bei der Handhabung dieser flüssigen Erdölfraktionen treten, insbesondere bei Unfällen, wie sie beispielsweise durch überhitzte Radlager oder auch bei Verkehrsunfällen von Straßenfahrzeugen auftreten können, flüssige Erdölderivate in die Umwelt. Treten diese Produkte direkt in den Wasserkreislauf ein, so kann durch Oberflächenbarrieren und gleichzeitiger Bindung der Öle oder Benzine mit entsprechenden Bindemitteln oder auch durch Absaugen der Oberfläche eine Kontamination von weiteren Gebieten im allgemeinen verhindert werden.

Treten die Erdölprodukte in Sande, Schotter oder Erdreich, so werden sie von diesen Substraten aufgenommen und bei Beaufschlagung mit Wasser langsam und schrittweise abgegeben. Eine derartig langfristige Abgabe der Erdölprodukte kann jedoch zur verstärkten Kontamination von Grundwasserströmen führen, wodurch Grundwasserresourcen nicht mehr Genußzwecken zugeführt werden können. Bei Unfällen mit Kontamination von Substraten wird dementsprechend in der Regel so vorgegangen, daß die kontaminierten Substrate ausgehoben werden und einer Sonderbehandlung zugeführt werden. Eine derartige Sonderbehandlung kann beispielsweise durch Extraktion durchgeführt werden, oder aber wird das kontaminierte Substrat einer thermischen Behandlung unterworfen, wobei die Erdölprodukte verbrennen. In beiden Fällen liegt anschließend ein steriles Substrat vor, das, so es beispielsweise der Landwirtschaft wieder zugeführt werden soll, belebt werden muß.

Es sind eine Reihe von Mikroorganismen bekannt, die in der Lage sind, flüssige oder auch gasförmige Kohlenwasserstoffe abzubauen und in Eiweiß umzuwandeln. Weiters ist bekannt, daß Mikroorganismen einem Adaptionsprozeß unterliegen und in der Lage sind, selbst steigende Kozentrationen sogar von toxischen Stoffen, wie Phenole u. dgl., ohne selbst Schaden zu nehmen, abzubauen. Auch die Mikroorganismen, welche als natürliche Mischung innerhalb von Substraten, z. B. Erdreich, Sanden, Schotter od. dgl. vorliegen, sind in der Lage, Erdölprodukte abzubauen. Der Abbau erfolgt jedoch so langsam, daß zusätzliche Maßnahmen erforderlich sind. Um die Abbaugeschwindigkeit zu erhöhen, ist bereits bekannt, dem mit Erdölprodukten kontaminierten Erdreich entweder Mischkulturen, wie sie beispielsweise in Traubentrestern vorliegen, oder auch Nährstoffe in Form von Kompost zuzusetzen. Bei diesen bekannten Verfahren ist es ebenfalls nachteilig, daß der Abbau relativ langsam erfolgt und weiters, daß mit sinkender Olkonzentration der Abbau so langsam ist, daß teilweise eine jahrelange Behandlung der kontaminierten Substrate erfolgen muß, um einen im wesentlichen zur Gänze vorliegenden Abbau des Erdöls zu erreichen.

Die vorliegende Erfindung hat sich zur Aufgabe gestellt, ein Verfahren zum Regenerieren von Mikroorganismen aufweisenden Substraten zu schaffen, das es erlaubt, mit den natürlich vorkommenden Mikroorganismen die, insbesondere bei Raumtemperatur, flüssigen Erdölprodukte od. dgl. abzubauen, so daß ein sowohl in der Zusammensetzung als auch mit der Mikroorganismenmischung ein natürliches Substrat, daß unmittelbar zum Einsatz, z. B. für Pflanzenbau od. dgl., kommen kann, erhalten wird. Ein weiteres Ziel der vorliegenden Erfindung besteht darin, den Abbau der Erdölprodukte im Substrat gegenüber den bekannten Verfahren zu beschleunigen. Ein weiteres Ziel der vorliegenden Erfindung besteht auch darin, eine möglichst geringe Endkonzentration der Erdölprodukte im Substrat in kurzer Zeit zu erreichen.

Das erfindungsgemäße Verfahren zum Regenerieren von Mikroorganismen aufweisenden Substraten, z. B. Erdreich, Sanden od. dgl., welche mit bis zu einer Temperatur von 80° C, insbesondere bei Raumtemperatur, flüssigen Erdölfraktionen, Erdölprodukten od. dgl., z. B. Heizöl, Dieselöl, insbesondere Kohlenwasserstoffen, kontaminiert sind, wobei das Substrat mit Nährstoffen vermischt und das Gemisch, insbesondere einer aeroben Fermentation unterzogen wird, besteht im wesentlichen darin, daß das kontaminierte Substrat mit vorzugsweise 0,1 Gew.-% bis 5,0 Gew.-%, insbesondere 0,5 Gew.-% bis 2,0 Gew.-%, sterilisierter Mikroorganismenmasse, bezogen auf die Mischung, vermischt wird, und daß während der Fermentation ein Wassergehalt von zumindest 5,0 Gew.-%, insbesondere zwischen 10,0 Gew.-% und 15,0 Gew.-%, eingehalten wird. Durch die Zugabe von sterilisierter Mikroorganismenmasse wird ein für das Wachstum der im Substrat natürlich vorkommenden Mikroorganismen besonders günstiges Nährstoffangebot gegeben, wobei durch die sterile Mikroorganismenmasse verhindert wird, daß das Wachstum der natürlich im Substrat vorkommenden Mikroorganismen gehemmt und von einem anderen Stamm dominiert wird. Durch die an sich geringen Konzentrationen von 0,1 Gew.-% bis 5,0 Gew.-% wird keine künstliche Verdünnung des kontaminierten Substrates durchgeführt, sondern es kommt lediglich zu einer geringfügigen Gesamtgewichtszunahme. Die bevorzugte Grenze von 0,5 Gew.-% bis 2,0 Gew.-% an steriler Mikroorganismenmasse optimiert einen unerwünschten Volumszuwachs gegenuber einem optimalen Angebot an Nährstoffen durch die sterile Mikroorganismenmasse. Wird während der Fermentation ein Wassergehalt von zumindest 5,0 Gew.-%, insbesondere zwischen 10,0 Gew.-% und 15,0 Gew.-%, eingehalten, so ist auf der einen Seite sichergestellt, daß die flüssigen Erdölfraktionen im Substrat verbleiben und auf der anderen Seite ein erwünschtes hohes Wachstum der Mikroorganismen sichergestellt ist.

Wird die Mikroorganismenmasse vor dem Vermischen mit dem Substrat wärmesterilisiert, insbesondere dampfsterilisiert, vorzugsweise bei mindestens 105° C, so ist einerseits sichergestellt, daß keine das Wachstum der Mikroorganismen im Substrat hemmenden Substanzen dem Substrat beim Vermischen zugeführt werden, wobei weiters durch die Wärmesterilisierung, insbesondere Dampfsterilisierung, bereits einen Aufschluß der Mikroorganismensubstanz erfolgt, so daß ein leichterer Zugang zu den Nährstoffen gegeben ist.

Wird als Mikroorganismenmasse, insbesondere extrahierte Mycelmasse, aus der Antibiotika-, insbesondere Penicillinherstellung, verwendet, so ist ein besonders vorzüglicher Nährstotf gegeben, der sich bereits als Zugabe am Erdreich, insbesondere zur Begrünung von Böschungen u. dgl., bewährt hat, wobei weiters ein besonders hoher Gehalt an Nährstoffen pro Gewichtseinheit vorliegt.

Wird als Mikroorganismenmasse sterilisierter Klärschlamm aus biologischen Abwasserkläranlagen verwendet, so liegt eine besonders hohe Vielfalt an unterschiedlichen Nährstoffen vor, wobei gleichzeitig der Klärschlamm ebenfalls einer biologisch zweckmäßigen Aufarbeitung zugeführt wird.

Wird ein Gemisch fermentiert, das mit Sand aufgebaut wird, wobei vorzugsweise ein Gewichtsverhältnis von Sand zu Substrat, insbesondere kontaminiertem Erdreich, von 1 : 4 bis 2 : 1 eingehalten wird, so kann eine besonders gute Verteilung der Mikroorganismenmasse aufgrund der partikulierten Struktur des Sandes erreicht werden, wobei weiters eine poröse Struktur, wie sie beispielsweise für die Zufuhr von Sauerstoff und Abfuhr von Gasen zweckdienlich ist, besonders einfach erreichbar ist.

Wird ein Gemisch mit höchstens 5,0 Gew.-%, insbesondere höchstens 2,5 Gew.-%, Erdölprodukten zu Beginn der Fermentation eingesetzt, so wird eine Schädigung der im Substrat vorliegenden natürlichen Mikroorganismen vermieden, wobei gleichzeitig eine Adaption der Mikroorganismen erreicht wird. Weiters kann aufgrund der relativ hohen Konzentration ein besonders schneller Abbau erreicht werden.

Wird während der Fermentation ein mit in einer höheren Konzentration als im Gemisch vorliegend, mit Erdölprodukten kontaminiertes Erdreich dem Gemisch zugesetzt, so können die bereits adaptierten Mikroorganismen für einen besonders raschen Abbau der Erdölprodukte dienen, wobei weiters eine junge Population von im natürlichen Substrat vorkommenden Mikroorganismen dem Gemisch zugefügt wird.

Wird vor der Zugabe von kontaminiertem Substrat zur bereits teilweise fermentierten Mischung ein Teil derselben abgezogen und insbesondere mit einer Mischung geringerer Konzentration an Erdölprodukten vermischt und fermentiert, so kann dadurch ein quasi Kontinuierliches Verfahren mit einzelnen Stufen erreicht werden, wobei jeweils sichergestellt ist, daß die Konzentration von einem Fermentationsort zum anderen Fermentationsort abnimmt und weiters die Gesamtmasse des zu behandelnden Substrates konstant bleibt, wobei am Beginn der Fermentation die höchste Konzentration und am Ende der Fermentation die niedrigste Konzentration an Erdölprodukten vorliegt, so daß jeweils der letzte Ort der Fermentation jeweils am Ende die erwünscht niedrigste Konzentration an Erdölprodukten aufweist.

Eine Beschleunigung der Fermentation kann dadurch erreicht werden, daß auch während der Fermentation sterilisierte Mikroorganismenmasse, vorzugsweise 0,1 Gew.-% bis 5,0 Gew.-%, insbesondere 0,5 Gew.-% bis 2,0 Gew.-%, bezogen auf die Mischung, erneut zugemischt wird.

Wird eine Mischung mit Strohhäcksel, Holzwolle od. dgl., fermentiert, so ist eine besonders gute Zu- und Abfuhr der Gase gewährleistet, wobei auch Zellulose abbauende Mikroorganismen im Substrat angereichert werden.

Wird die Mischung während der Fermentation in der Atmosphäre umgewälzt, so ist ein besonders vorteilhafter aerober Fermentationsprozeß gewährleistet.

Im folgenden wird die Erfindung anhand der Beispiele und der Tabellen näher erläutert.

### Beispiel 1:

12 kg Gärtnererde aus einer Gärtnerei in Gänserndorf wurden mit 6 kg Sand aus einer Schottergrube in Gänserndorf mit einer Korngröße zwischen 0,5 mm bis 2,0 mm in einem herkömmlichen Zwangsmischer vermischt. 324 g dieser Mischung wurden durch Heizöl extraleicht mit einem Siedebereich von 180° C bis 360° C aus der Produktion der ÖMV, Raffinerie Schwechat, versetzt, wonach die Mischung erneut im Zwangsmischer vermischt wurde, so daß eine KohlenwasserstoffKonzentration von 1,8 Gew.-% (bezogen auf Trockensubstanz) eingestellt wurde. Der Wassergehalt der Mischung wurde auf 15 Gew.-% eingestellt. Drei Mischungen obiger Art wurden in einer auf 22° C temperierten Halle in drei oben offenen Behältern unter wöchentlicher Umwälzung gelagert. Die in den Tabellen angegebenen Werte stellen, so wie in den folgenden Beispielen, jeweils das arithmetische Mittel von drei gemessenen Werten dar. Es wurden monatlich Proben entnommen und der Gehalt an Heizol extraleicht ermittelt.

Der Wassergehalt wurde, so wie in den folgenden Mischungen, jeweils zu Beginn einer Woche auf 15 Gew.-% eingestellt und betrug am Ende der Woche zwischen 8 Gew.-% und 12 Gew.-%. Wie den Tabellen zu entnehmen, tritt in den ersten beiden Monaten eine Abnahme des Ölgehaltes um ca. jeweils 0,2 Gew.-% ein, wohingegen in späterer Folge die Abnahme auf ca. 0,1 Gew.-% pro Monat sich verringert, wohingegen ab dem 8. Monat eine außerordentlich geringe Abnahme des Ölgehaltes eintritt.

### Beispiel 2:

Der Mischung gemäß Beispiel 1 wurde ein Pilzmyzel, das ein Nebenprodukt der Penicillinherstellung mit Penicillium chrysogenum der Firma Biochemie Kundl in Tirol anfällt und unter der Markenbezeichnung "Biosol" auf den Markt gebracht wird, zugemischt. Die Biomasse wurde bei einer Temperatur über 105° C getrocknet. Die Mischung mit der Mikroorganismenmasse wurde so eingestellt, daß ein Heizölgehalt von 1,8 Gew.-% und eine Mikroorganismenmasse von 0,75 Gew.-% erhalten wurde. Der Gehalt an Heizöl im Substrat nahm im ersten Monat besonders stark ab, u. zw. ca. auf 0,6 Gew.-%, wobei die anschließende Abnahme des Heizölgehaltes bis auf 0,08 Gew.-% nach 12 Monaten, also ca. 1/7 des Wertes der unbehandelten Probe, betragen hatte.

### Beispiel 3:

Es wurde eine Probe gemäß Beispiel 2 hergestellt, wobei anstelle des Pilzmyceles eine sterilisierte Bakterienmasse der Abwasserreinigungsanlage (Überschußschlamm) aus der Penicillinherstellung der Firma Biochemie Kundl in Tirol (Markenbezeichnung "Bactosol") verwendet wurde. Diese Bakterienmasse bewirkte in den ersten Monaten einen rascheren Abbau, wonach aber in etwa gleiche Endwerte wie in Beispiel 2 erhalten wurden.

### Beispiel 4:

Einer Mischung gemäß Beispiel 2 wurden 50 g Strohhäcksel zugemischt, wodurch in der anfänglichen Fermentation ein besserer Abbau erreicht wird. Die Endwerte sind jedoch analog Beispiel 2.

### Beispiel 5:

Der Mischung gemäß Beispiel 1 wurden 50 g Strohhäcksel untermischt, wobei in der Anfangsphase eine bessere Fermentation erreicht werden konnte. Die Endwerte entsprechen allerdings ebenfalls den Endwerten des Beispiels 1, bei welcher Mischung kein Strohhäcksel vorliegt.

### Beispiel 6:

Eine Mischung gemäß Beispiel 1 wurde mit fermentierten Traubentrester versetzt, so daß die Endmischung 2,5 Gew.-% Traubentrester und 1,8 Gew.-% Heizöl extraleicht aufwies. Der Abbau des Erdölderivates war anfangs wesentlich schneller als im Beispiel 1, wobei allerdings die Endkonzentration nach 12 Monaten etwa den 5-fachen Wert der erfindungsgemäßen Beispiele aufwies.

### Beispiel 7:

Es wurde analog Beispiel 6 verfahren, wobei die Konzentration der fermentierten Traubentrester 1,0 Gew.-% betrug. Der anfängliche Abbau der Erdölderivate war langsamer, wohingegen die Endkonzentration nach 12 Monaten entsprechend dem Beispiel 6 war.

### Beispiel 8:

Eine Mischung gemäß Beispiel 1 wurde mit Kompost und Rinde im Gewichtsverhältnis 1 : 1 versetzt, so daß eine Endkonzentration von 2,5 Gew.-% in dieser Mischung vorlag, wobei weiters die Konzentration des Erdölderivates auf 1,8 Gew.-% eingestellt wurde. Die Abnahme des Erdölproduktgehaltes hat im wesentlichen dem des Beispieles 1, also der unbehandelten Mischung, entsprochen.

### Beispiel 9:

Es wurde eine Mischung gemäß Beispiel 1 angefertigt, wobei 1,0 Gew.-% in der Mischung 1 : 1 aus Kompost und Rinde vorgelegen ist. Die Werte unterscheiden sich nicht wesentlich von jenen des Beispieles 8. Die Endkonzentration betrug wie dort ca. 0,6 Gew.-%.

### Beispiel 10:

Eine Mischung gemäß Beispiel 1 wurde mit 3,1 Gew.-% einer sterilisierten Klärschlammbiomasse aus einer städtischen biologischen Abwasserkläranlage versetzt. Die Konzentrationsabnahme des Erdölderivates war in den ersten beiden Monaten geringer als bei den Beispielen 2, 3, und 4, führte jedoch bereits im 5. Monat in etwa zum selben Gehalt.

### Beispiel 11:

Der Mischung gemäß Beispiel 2 wurden sowohl nach dem 2. Monat als auch nach dem 4. Monat jeweils 0,75 Gew.-% des im Beispiel 2 angeführten Pilzmycels zugeführt. Wie den Zahlenwerten zu entnehmen, ist durch die weitere Zugabe des Pilzmycels eine drastische Abnahme des Ölgehaltes zu verzeichnen, wobei in ca. der Hälfte der Zeit die gleiche absolute Abnahme des Erdölgehaltes erreicht werden konnte.

### Beispiel 12:

Einer Mischung gemäß Beispiel 3 wurden nach 2 Monaten und nach 4 Monaten jeweils 0,75 Gew.-% der im Beispiel 3 angeführten Bakterienmasse zugegeben, wobei Analogwerte wie im Beispiel 11 erhalten wurden.

Die Mengenangaben in den Tabellen sind ppm Erdölprodukte in Trockensubstanz der Mischung.

Der Erdölproduktgehalt wurde Lm Tetrachlorkohlenstoffextrakt nach der Infrarotmethode bestimmt.

Die Erfindung wurde anhand von Beispielen erläutert, wobei als Erdölfraktion Heizöl extraleicht zum Einsatz kam. Systematische Versuche mit anderen Erdölderivaten, wie Dieselkraftstoff, Heizöl leicht und Heizöl schwer, wobei auch Konzentrationen bis 5,0 Gew.-% an steriler Mikroorganismenmasse als auch bis zu 6,0 Gew.-% an Erdölprodukten zum Einsatz kamen, haben analoge Resultate gezeigt. Anstelle des Pilzmyceles und Bakterienmasse aus der Penicillinherstellung bzw. Klärschlamm aus biologischen Abwasserklaranlagen wurden auch Hefen, Kefirhefen erprobt, wobei entsprechende Werte erhalten wurden.

**Tabelle I**

| Beispiel | 1 | 2 | 3 | 4 | 5 | 6 |
|---|---|---|---|---|---|---|
| ppm nach Monaten | | | | | | |
| 1 | 15.750 | 6.320 | 5.400 | 6.120 | 15.500 | 12.650 |
| 2 | 12.000 | 5.750 | 4.450 | 4.300 | 11.800 | 10.500 |
| 3 | 10.800 | 4.020 | 3.500 | 3.510 | 10.750 | 8.650 |
| 4 | 10.000 | 3.700 | 3.330 | 3.300 | 9.900 | 7.550 |
| 5 | 8.650 | 3.030 | 3.100 | 3.050 | 8.580 | 6.090 |
| 6 | 7.900 | 2.000 | 2.080 | 2.100 | 7.880 | 5.040 |
| 7 | 7.350 | 1.800 | 1.900 | 1.870 | 7.400 | 4.680 |
| 8 | 7.070 | 1.210 | 1.310 | 1.320 | 7.000 | 4.510 |
| 9 | 6.820 | 900 | 950 | 1.010 | 6.810 | 4.500 |
| 10 | 6.670 | 850 | 850 | 870 | 6.630 | 4.000 |
| 11 | 6.100 | 830 | 830 | 840 | 6.100 | 3.900 |
| 12 | 6.020 | 800 | 800 | 810 | 6.010 | 3.850 |

**Tabelle II**

| Beispiel | 7 | 8 | 9 | 10 | 11 | 12 |
|---|---|---|---|---|---|---|
| ppm nach Monaten | | | | | | |
| 1 | 12.900 | 16.020 | 15.090 | 8.300 | 6.320 | 5.400 |
| 2 | 10.850 | 11.900 | 11.900 | 6.420 | 5.750 | 4.450 |
| 3 | 8.970 | 10.880 | 10.600 | 4.700 | 3.520 | 3.020 |
| 4 | 7.850 | 9.900 | 10.000 | 2.980 | 3.300 | 2.900 |
| 5 | 6.190 | 8.700 | 8.750 | 2.900 | 1.950 | 2.020 |
| 6 | 5.140 | 7.950 | 8.000 | 2.100 | 900 | 1.050 |
| 7 | 4.720 | 7.530 | 7.400 | 2.000 | | |
| 8 | 4.590 | 7.120 | 6.970 | 1.300 | | |
| 9 | 4.300 | 6.900 | 6.850 | 950 | | |
| 10 | 4.090 | 6.590 | 6.690 | 850 | | |
| 11 | 3.920 | 6.110 | 6.120 | 840 | | |
| 12 | 3.870 | 6.030 | 6.010 | 810 | | |

## Patentansprüche

1. Verfahren zum Regenerieren von Mikroorganismen aufweisenden Substraten, z. B. Erdreich, Sanden od. dgl., welche mit bis zu einer Temperatur von 80° C, insbesondere bei Raumtemperatur, flüssigen Erdölprodukten, Erdölfraktionen od. dgl., z. B. Heizöle, Dieselöle, insbesondere Kohlenwasserstoffen, kontaminiert sind, wobei das Substrat mit Nährstoffen vermischt, und das Gemisch einer, insbesondere aeroben, Fermentation unterzogen wird, dadurch gekennzeichnet, daß das kontaminierte Substrat mit, vorzugsweise 0,1 Gew.-% bis 5,0 Gew.-%, insbesondere 0,5 Gew.-% bis 2,0 Gew.-%, sterilisierter Mikroorganismenmasse, bezogen auf die Mischung, vermischt wird, und daß während der Fermentation ein Wassergehalt von zumindest 5,0 Gew.-%, insbesondere zwischen 10,0 Gew.-% und 15,0 Gew.-%, eingehalten wird.

2. Verfahren nach Anspruch 1, dadurch gekennzeichnet, daß die Mikroorganismenmasse vor dem Vermischen mit dem Substrat wärmesterilisiert, insbesondere dampfsterilisiert, vorzugsweise bei mindestens 105° C, wird.

3. Verfahren nach Anspruch 1 oder 2, dadurch gekennzeichnet, daß als Mikroorganismenmasse, insbesondere extrahierte, Mycelmasse aus der Antibiotika-, insbesondere Penicillinherstellung, verwendet wird.

4. Verfahren nach Anspruch 1, 2 oder 3, dadurch gekennzeichnet, daß als Mikroorganismenmasse sterilisierter Klärschlamm aus biologischen Abwasserkläranlagen verwendet wird.

5. Verfahren nach einem der Anspruche 1 bis 4, dadurch gekennzeichnet, daß ein Gemisch fermentiert wird, das mit Sand aufgebaut wird, wobei vorzugsweise ein Gewichtsverhältnis von Sand zu Substrat, insbesondere kontaminiertem Erdreich, von 1 : 4 bis 2 : 1 eingehalten wird.

6. Verfahren nach einem der Ansprüche 1 bis 5, dadurch gekennzeichnet, daß ein Gemisch mit höchstens 5,0 Gew.-%, insbesondere 2,5 Gew.-%, Erdölprodukten zu Beginn der Fermentation eingesetzt wird.

7. Verfahren nach einem der Ansprüche 1 bis 6, dadurch gekennzeichnet, daß während der Fermentation ein mit Erdölprodukten kontaminiertes Erdreich in einer höheren Konzentration als im Gemisch vorliegt, diesem Gemisch zugesetzt wird.

8. Verfahren nach Anspruch 7, dadurch gekennzeichnet, daß vor Zugabe von kontaminiertem Substrat zur bereits teilweise fermentierten Mischung ein Teil desselben abgezogen wird, und insbesondere mit einer Mischung geringerer Konzentration an Erdölprodukten vermischt und fermentiert wird.

9. Verfahren nach einem der Ansprüche 1 bis 8, dadurch gekennzeichnet, daß auch wahrend der Fermentation, vorzugsweise in einer Menge von 0,1 Gew.-% bis 5,0 Gew.-%, insbesondere 0,5 Gew.-% bis 2,0 Gew-%, bezogen auf die Mischung, sterile Mikroorganismenmasse zugemischt wird.

10. Verfahren nach einem der Ansprüche 1 bis 9, dadurch gekennzeichnet, daß eine Mischung mit Strohhäcksel, Holzwolle od. dgl., fermentiert wird.

11. Verfahren nach einem der Ansprüche 1 bis 10, dadurch gekennzeichnet, daß die Mischung während der Fermentierung unter Luftzufuhr umgewälzt wird.

## Claims

1. Process for regenerating substrates containing microorganisms, e.g. soil, sands or the like, which are contaminated by liquid mineral oil products, mineral oil fractions or the like liquid up to a temperature of 80°C, in particular at room temperature, e.g. fuel oils, diesel oils, in particular hydrocarbons, in which the substrate is mixed with nutrients and the mixture is subjected to an, in particular, aerobic fermentation, characterized in that the contaminated substrate is mixed with preferably 0.1 % by weight to 5.0 % by weight, in particular 0.5 % by weight to 2.0 % by weight, of sterilized microorganism mass, based on the mixture, and in that during the fermentation a water content of at least 5.0 % by weight, in particular between 10.0 % by weight and 15.0 % by weight, is maintained.

2. Process according to Claim 1, characterized in that the microorganism mass, before being mixed with the substrate, is heat sterilized, in particular steam sterilized, preferably at at least 105°C.

3. Process according to Claim 1 or 2, characterized in that the microorganism mass used is, in particular extracted, mycelium mass from antibiotic production, in particular penicillin production.

4. Process according to Claim 1, 2 or 3, characterized in that the microorganism mass used is sterilized sewage sludge from biological waste water treatment plants.

5. Process according to one of Claims 1 to 4, characterized in that a mixture is fermented which is made up with sand, a weight ratio of sand to substrate, in particular contaminated soil, of 1:4 to 2:1 preferably being maintained.

6. Process according to one of Claims 1 to 5, characterized in that a mixture having at most 5.0 % by weight, in particular 2.5 % by weight, of mineral oil products at the beginning of the fermentation is used.

7. Process according to one of Claims 1 to 6, characterized in that, during the fermentation, a soil contaminated by mineral oil products at a higher concentration than in the mixture is added to this mixture.

8. Process according to Claim 7, characterized in that, prior to addition of contaminated substrate to the already partly fermented mixture, part of the same is taken off and is mixed and fermented, in particular with a mixture of lower concentration of mineral oil products.

9. Process according to one of Claims 1 to 8, characterized in that sterile microorganism mass is also added during the fermentation, preferably in an amount of 0.1 % by weight to 5.0 % by weight, in particular 0.5 % by weight to 2.0 % by weight, based on the mixture.

10. Process according to one of Claims 1 to 9, characterized in that a mixture containing chopped straw, wood wool or the like is fermented.

11. Process according to one of Claims 1 to 10, characterized in that the mixture is circulated with air feed during the fermentation.

## Revendications

1. Procédé de régénération de substrats présentant des micro-organismes, par exemple de la terre, des sables ou des matières analogues, qui sont contaminés par des produits pétroliers, fractions de pétrole ou matières analogues qui sont liquides jusqu'à une température de 80°C, en particulier à la température ambiante, par exemple des fuels, des carburants Diesel, en particulier des hydrocarbures, le substrat étant mélangé à des substances nutritives et le mélange étant soumis à une fermentation, en particulier en aérobie, caractérisé en ce que le substrat contaminé est mélangé à de préférence 0,1 % en poids à 5,0 % en poids, en particulier 0,5 % en poids à 2,0 % en poids, par rapport au mélange, d'une masse stérilisée de micro-organismes et en ce que, pendant la fermentation, une teneur en eau d'au moins 5,0 % en poids, en particulier entre 10,0 % en poids et 15,0 % en poids, est maintenue.

2. Procédé suivant la revendication 1, caractérisé en ce que la masse de micro-organismes est, avant le mélange avec le substrat, stérilisée à chaud, en particulier par de la vapeur, de préférence à au moins 105°C.

3. Procédé suivant l'une des revendications 1 et 2, caractérisé en ce que, comme masse de micro-organismes, on utilise en particulier une masse de mycélium extraite, issue de la préparation d'antibiotiques, en particulier de pénicilline.

4. Procédé suivant l'une des revendications 1 à 3, caractérisé en ce que, comme masse de microorganismes, on utilise des boues de curage stérilisées provenant d'installations de clarification biologique des eaux résiduaires.

5. Procédé suivant l'une des revendications 1 à 4, caractérisé en ce qu'on fait fermenter un mélange qui est constitué avec du sable, un rapport pondéral entre le sable et le substrat, en particulier la terre contaminée, de 1/4 à 2/1 étant de préférence respecté.

6. Procédé suivant l'une des revendications 1 à 5, caractérisé en ce qu'on met en oeuvre au commencement de la fermentation un mélange avec au maximum 5,0 % en poids, en particulier 2,5 % en poids, de produits pétroliers.

7. Procédé suivant l'une des revendications 1 à 6, caractérisé en ce que, pendant la fermentation, une terre contaminée par des produits pétroliers en une concentration supérieure à celle présente dans le mélange est ajoutée à ce mélange.

8. Procédé suivant la revendication 7, caractérisé en ce que, avant l'addition de substrat contaminé au mélange déjà partiellement fermenté, une partie de ce dernier est soutirée et est mélangée et mise à fermenter en particulier avec un mélange ayant une concentration plus faible en produits pétroliers.

9. Procédé suivant l'une des revendications 1 à 8, caractérisé en ce que, également pendant la fermentation, on ajoute une masse de micro-organismes stérile de préférence en une quantité de 0,1 % en poids à 5,0 % en poids, en particulier de 0,5 % en poids à 2,0 % en poids, par rapport au mélange.

10. Procédé suivant l'une des revendications 1 à 9, caractérisé en ce qu'un mélange est mis à fermenter avec de la paille hachée, de la laine de bois ou une matière analogue.

11. Procédé suivant l'une des revendications 1 à 10, caractérisé en ce que le mélange est, pendant la fermentation, mis en circulation avec apport d'air.
